# EUROPÄISCHE PATENTSCHRIFT

(11) **EP 2 994 085 B1**
(45) Veröffentlichungstag und Bekanntmachung des Hinweises auf die Patenterteilung: **12.07.2017**
(21) Anmeldenummer: 14730732.6
(22) Anmeldetag: 06.05.2014
(51) Int. Cl.: A61F 13/84

(54) **VORRICHTUNG UMFASSEND EINEN ABSORPTIONSARTIKEL UND EINE ENTLÜFTUNGSVORRICHTUNG UND ARBEITSVERFAHREN UNTER VERWENDUNG DER ENTLÜFTUNGSVORRICHTUNG ZUR BEEINFLUSSUNG DES FEUCHTWARMEN KLIMAS IN EINEM ABSORPTIONSARTIKEL**
DEVICE COMPRISING AN ABSORPTION ARTICLE AND AN AIR-EXTRACTION DEVICE, AND METHOD OF OPERATION USING THE AIR-EXTRACTION DEVICE FOR INFLUENCING THE HUMID CONDITIONS IN AN ABSORPTION ARTICLE
DISPOSITIF COMPRENANT UN ARTICLE ABSORBANT ET UN DISPOSITIF D'AÉRATION, ET PROCÉDÉ DE TRAVAIL REPOSANT SUR L'UTILISATION DU DISPOSITIF D'AÉRATION POUR INFLUER SUR LES CONDITIONS DE TEMPÉRATURE ET D'HUMIDITÉ DANS UN ARTICLE ABSORBANT

(30) Priorität: 06.05.2013 DE 102013104659
(43) Veröffentlichungstag der Anmeldung: 16.03.2016
(73) Patentinhaber: Eberhard Timm GmbH, 21614 Buxtehude (DE)
(72) Erfinder: TIMM, Eberhard, 21614 Buxtehude (DE)
(74) Vertreter: Müller Schupfner & Partner Patent- und Rechtsanwaltspartnerschaft mbB
(86) Internationale Anmeldenummer: PCT/DE2014/000229
(87) Internationale Veröffentlichungsnummer: WO 2014/180461

(56) Entgegenhaltungen:
- WO-A1-03/053300
- WO-A1-2011/122604
- US-A1- 2002 177 834
- US-A1- 2004 059 311

## Beschreibung

Die Erfindung bezieht sich auf eine Vorrichtung umfassend einen Absorptionsartikel und eine Entlüftungsvorrichtung für das feuchtwarme Klima, das im Bereich eines an einem Patienten angelegten Absorptionsartikels, wie beispielsweise einer Windel oder Binde, vorherrscht und ein Arbeitsverfahren zur Verbesserung des Klimas in dem Absorptionsartikel durch Luftabsaugen aus dem Absorptionsartikel mit der Entlüftungsvorrichtung.

Absorptionsartikel in Form von Windeln, Windelhosen oder Binden, die eine Absorptionssubstanz, wie einen Superabsorber, enthalten, sind vielfach bekannt. Zum Einsatz kommen Windeln z.B. bei Babys oder Kleinkindern und krankheitsbedingt bei Patienten in der Alten- und Krankenpflege. Vereinfachend wird eine Harn- oder eine Stuhlinkontinenz oder das Unvermögen aus anderen Gründen eine Toilette nicht mehr selbständig rechtzeitig aufsuchen zu können, zusammenfassend vorliegend als Inkontinenz bezeichnet. Bei Patienten, die an Inkontinenz leiden und krankheitsbedingt die Windel selbst nicht wechseln können, z.B. weil sie das Bett nicht mehr alleine verlassen können, kommt es häufig zu Dekubitus-Geschwüren. Diese Druck- oder Wundliegegeschwüre entstehen insbesondere in der Kreuzbeinregion und an den Oberschenkelknochen infolge der Druckbelastung bei Bettlägerigkeit oder eines lang anhaltenden Aufenthalts in Rollstühlen. Wenn das Tragen der bei Inkontinenz unverzichtbaren Windeln auch das Entstehen der Geschwüre nicht direkt verursacht, so sind sie doch dabei förderlich und wirken sich bei der Wundbehandlung negativ aus. Gründe dafür sind die hohen Temperaturen in unphysiologischen Bereichen und die starke Feuchtigkeit, die durch Schwitzen und aus den Ausscheidungen bei Harn- und Stuhlinkontinenz mit hohen sauren pH-Werten entstehen.

Aus der US 5515543 ist es bekannt, Bekleidung, wie eine Jacke, durch Einblasen von Luft innen zu belüften. Umgekehrt betrifft die US 5192276 A eine Vorrichtung zum Absaugen von Rauchgasen während einer Elektrokauterisation in Form eines flachen, flexiblen und plattenförmigen Hohlkörpers mit einer Vielzahl von Öffnungen auf der Oberseite und einem Klebstoff zur Befestigung unmittelbar auf der Haut eines Patienten auf der Unterseite.

Stand der Technik, der sich mit dem Absaugen von Urin auseinandersetzt, ist sehr vielfältig bekannt. Die US 8241262 B2 offenbart z.B. ein flexibles Kissen zum Sammeln und Transportieren von Flüssigkeit zu einem Sammelpunkt, das unter einem inkontinenten Patienten positioniert oder in der Unterwäsche getragen werden kann. Die WO 2006/014240 A2 beschreibt eine Urin-Absaug- und Auffangeinrichtung. Urin wird körpernah aufgesaugt und dann in einer Urin-Auffangeinrichtung hineingesaugt. Ein weitere Vorrichtung zum Aufnehmen und Ableiten von Urin ist aus der DE 4236097 A1 bekannt, allerdings ohne Absaugvorrichtung. Die US 7018366 B2 betrifft ähnlich wie die WO 2006/014240 A2 eine Vorrichtung zur Aufnahme von Urin. Das wie eine Windel oder Unterwäsche geformte Kleidungsstück umfasst einen Ansaugkrümmer und eine Anzahl von perforierten Rohren sandwichartig zwischen Materialschichten. Gelangt Urin in das Kleidungsstück, wird der Urin durch die Löcher mittels Unterdruck aufgesaugt und über einen Schlauch in einen Beutel gepumpt. Obigem Stand der Technik, der sich mit dem Absaugen oder Ableiten von Urin auseinandersetzt, fehlt es an einem Absorptionsartikel zur Aufnahme von Flüssigkeit und es besteht dort die Aufgabe Flüssigkeit aufzusaugen und nicht wie in der vorliegenden Erfindung Luft.

Die US 2002/177834 A1 offenbart einen Absorptionsartikel, in den eine Belüftungsvorrichtung untrennbar integriert ist, in deren Hohlraum Luft hineingepumpt wird und die Luft über ventilartig ausgeprägte Öffnungen nach außen in den Absorptionsartikel einbläst.

WO 2011/122604 A1 offenbart eine Windel, in die ein Luftleitsystems eingebaut ist. Das Luftleitsystem dient der Belüftung der Windel über Löcher und ist als integraler Bestandteil der Windel ausgebildet. Eine Pumpe ist nicht anschließbar.

Es ist Aufgabe der Erfindung, den Einsatz von Absorptionsartikeln, wie Windeln, derart zu verbessern bzw. zu ergänzen, dass das Klima im Bereich der betroffenen Hautbereiche in Bezug auf Feuchtigkeit und Temperatur hautgünstig beeinflusst wird, um damit die Gefahr der Bildung von Dekubitus-Geschwüren zu mindern, die Heilung zu begünstigen bzw. die Beeinträchtigung durch diese zu lindern.

Die gestellte Aufgabe ist erfindungsgemäß durch den Gegenstand des Anspruchs 1 gelöst. Vorteilhafte Ausführungsformen sind Gegenstand der Unteransprüche oder nachfolgend beschrieben.

Die erfindungsgemäße Vorrichtung umfasst einen Absorptionsartikel, eine Pumpe und eine Entlüftungsvorrichtung in, unter oder auf dem Absorptionsartikel, wobei die Entlüftungsvorrichtung ein flexibles Behältnis, einen luftleitenden Innenraum, einen Anschluss, und eine Oberfläche mit luftundurchlässigen Zonen und luftdurchlässigen Zonen in Form von Durchbrechungen umfasst, um Luft aus dem Absorptionsartikel durch die Durchbrechungen über den Anschluss in den Innenraum (3a) des Behältnisses abzusaugen und die Entlüftungsvorrichtung eine separat handhabbare Einheit ist und in den Absorptionsartikel eingeschoben oder ein- bzw. aufgelegt ist, wobei die Entlüftungsvorrichtung über den Anschluss mit der Pumpe verbunden ist und die Pumpe eine Pumpe im Saugbetrieb ist, damit die belastete Luft aus dem Behältnis über den Anschluss durch Absaugen nach außen abführbar ist.

Die Erfindung betrifft weiter ein Arbeitsverfahren zur Beeinflussung des feucht-warmen Klimas im Bereich eines am Körper eines Patienten anliegenden Absorptionsartikels, wie beispielsweise einer Windel, umfassend das Absaugen von feucht-warmer, belasteter Luft aus dem Absorptionsartikel mittels einer als eine separat handhabbare Einheit ausgeführten Entlüftungsvorrichtung im, unter oder auf dem Absorptionsartikel umfassend einen von einem flexiblen Behältnis gebildeten luftleitenden Innenraum mit luftundurchlässigen und luftdurchlässigen Zonen und einem Anschluss, wobei belastete Luft aus dem Klimabereich des-Absorptionsartikels durch die luftdurchlässigen Zonen in den Innenraum des Behältnisses und über den Anschluss mit einer Pumpe im Saugbetrieb abgesaugt wird, damit die belastete Luft aus dem Behältnis über den Anschluss durch Absaugen nach außen abführbar ist.

Die in Verbindung mit dem Absorptionsartikel eingesetzte Entlüftungsvorrichtung sorgt dafür, dass aus dem feucht-warmen Klimabereich am Körper des Patienten belastete Luft abgesaugt und dann nach außen abgeführt wird. Das nachfließende Luftvolumen hat darüber hinaus einen kühlenden Effekt. Dazu weist die Entlüftungsvorrichtung ein Luftleitsystem auf, um über eine Fläche verteilt längs des Luftleitsystems aus dem feucht-warmen Klimabereich am Körper des Patienten belastete Luft einzusaugen und dann nach außen abzuführen.

Die Entlüftungsvorrichtung wird in den Absorptionsartikel eingeschoben oder ein- bzw. aufgelegt.

Die Entlüftungsvorrichtung soll keine Flüssigkeiten ansaugen, abgesehen ggf. von Kondensat aus der Luft, wie es beim Ansaugen von Luft anfallen könnte. Es ist aber bevorzugt, dass keine Kondensation in der Entlüftungsvorrichtung erfolgt oder wenn diese erfolgt, die Flüssigkeit von nachfolgender nicht vollständig gesättigter Luft aufgenommen wird, so dass über die Betriebszeit gesehen, keine Flüssigkeit in die Entlüftungsvorrichtung gelangt. Damit zuverlässig das Ansaugen von Flüssigkeit verhindert wird, wird die Entlüftungsvorrichtung und der Absorptionsartikel an einen Körper insbesondere so angelegt und relativ zueinander angeordnet, dass die Durchbrechungen der Entlüftungsvorrichtung sich in Lotrichtung über (nur der Höhe nach) den Orten wo Stuhlgang und/oder Urin im Absorptionsartikel aufgefangen wird, befinden, insbesondere in Sitz- und Liegeposition, um ein Aufsaugen von Flüssigkeit in die Entlüftungsvorrichtung zu verhindern, die entweder unmittelbar am Ort des Austretens vom Absorptionsartikel aufgenommen wird oder der Schwerkraft folgt und sich damit von den Durchbrechungen entfernt.

Der Absorptionsartikel ist insbesondere eine Windel, auch in Form einer Windelhose, oder eine Binde. Solche Windeln können z.B. als im Schritt umklappbares Flächengebilde mit verbindenden Seitenteilen oder als Windelhose in Slip-Form ausgebildet sein. Der Absorptionsartikel enthält vorzugsweise einen Superabsorber, der polare Flüssigkeiten, wie Wasser oder Urin, aufnimmt. Als Superabsorber werden insbesondere Polymere bezeichnet, die in der Lage sind, ein Vielfaches ihres Eigengewichts an polaren Flüssigkeiten aufzusaugen. Dies sind vor allem Wasser bzw. wässrige Lösungen. Bei der Aufnahme der Flüssigkeit quillt der Superabsorber auf und bildet ein Hydrogel.

Neben der Betriebsart "Absaugen" ist es auch möglich zeitweilig in eine andere Betriebsart zu wechseln und Luft in die Entlüftungsvorrichtung einzublasen, um die belastete Luft im feucht-warmen Klimabereich am Körper des Patienten zu verdrängen und so abzuführen. Dann werden beide Betriebsarten Absaugen und Einblasen kombiniert, z.B. zeitlich versetzt und intervallartig. Es ist allerdings bevorzugt für das Absaugen in der Summe längere Betriebszeiten als für das Einblasen der Luft vorzusehen.

Die Entlüftungsvorrichtung weist ein Luftleitsystem auf, um flächig längs des Luftleitsystems aus dem feucht-warmen Klimabereich am Körper des Patienten belastete Luft einzusaugen bzw. in den feucht-warmen Klimabereich einzublasen und dann nach außen abzuführen.

Durch das kontinuierliche Absaugen von Luft aus dem Inneren des Absorptionsartikels wird das Luftvolumen im Inneren des Absorptionsartikels ständig durch unbelastete, kühlere Luft von außen ersetzt, z.B. von vorgewärmter Luft aus dem Bettinneren. Es wird der Windel Stauwärme und Feuchtigkeit, vorzugsweise insbesondere in den körpernahen Bereichen des Absorptionsartikels, entzogen und der belastete Bereich wird abgekühlt. Mit Hilfe der Entlüftungsvorrichtung wird eine spürbare Klimaverbesserung in den betroffenen Bereichen erreicht. Das Klima im Windelbereich am Körper wird so von schädlichen Begleiterscheinungen wie übermäßige Luftfeuchte und Wärme befreit und die Entlüftungsvorrichtung kann somit einen Beitrag zur Vermeidung und Behandlung von Dekubitus-Geschwüren leisten.

Die Entlüftungsvorrichtung verfügt neben dem Abtransport von Luftfeuchtigkeit noch über eine weitere im Vordergrund stehende Funktion. Sie kann die Temperatur nahe der Hautoberfläche reduzieren. Dies ist insbesondere im Kreuzbeinbereich und im Innenbereich der Oberschenkel, wo sich die Temperatur bei Rücken- und Seitenlage des Patienten staut und über der normalen Körpertemperatur liegt. Die kühlere Luft, die die Entlüftungsvorrichtung vom Lufteintritt bis zum Abführen über den Schlauchanschluss durchströmt, baut die hohe Temperatur ab oder lässt sie gar nicht erst entstehen.

Die Entlüftungsvorrichtung umfasst ein flexibles Behältnis mit einem Anschluss für eine Pumpe, einen luftleitenden Innenraum und eine im Wesentlichen luftundurchlässige Oberfläche mit im Wesentlichen luftundurchlässigen Zonen und luftdurchlässigen Zonen mit Durchbrechungen, um Luft aus der Umgebung durch die Durchbrechungen in den Innenraum des Behältnisses und über den Anschluss abzusaugen. Die Durchbrechungen stellen Eintrittsflächen für Luft bereit. Der Innenraum wird vorzugsweise zumindest teilweise von Füllmaterial ausgefüllt. Die im Wesentlichen luftundurchlässige Oberfläche ist z.B. eine Folie, etwa eine Kunststofffolie.

Das flächige flexible Behältnis kann als Beutel mit Folienoberbahn und Folienunterbahn, zwischen denen sich vorzugsweise ein Füllmaterial befindet, ausgebildet sein. Der Beutel bzw. das Behältnis kann auch die Form eines Stabs, eines Schlauches oder eines mehrfach verzweigten Schlauches haben.

Das Füllmaterial dient zum Leiten der Luft und zum Beabstanden der Folienoberbahn und Folienunterbahn. Vorzugsweise sind Folienoberbahn und Folienunterbahn im Wesentlichen parallel zueinander angeordnet. Die zwei im Wesentlichen luftundurchlässigen Folienbahnen können auch aus einer Folienbahn gebildet sein, die an einer Kante umgeschlagen ist. Eine oder beide der im Wesentlichen luftundurchlässigen Folienbahnen weisen Durchbrüche für den Lufteintritt oder Luftaustritt auf. Dies schließt ein, dass sich die Durchbrüche im Randbereich der Folienbahnen befinden, wo die Folienbahnen zusammengefügt sind. Die Durchbrüche können in Zahl und Form unterschiedlich sein und nur auf der Außenfläche (vom Körper weg) oder nur auf der Innenseite (zum Körper hin) oder auf beiden Seiten gleichzeitig vorgesehen sein.

Nach einer weiteren Ausgestaltung der Erfindung ist vorgesehen, dass die Randverbindungen der Folienbahnen und ggf. auch die Rippen, an denen die Folienbahnen und die darüber befindliche Gewebe- bzw. Vliesschicht miteinander verbunden sind, durch ein Verkleben oder Verschweißen gebildet sind.

Nach einer weiteren Ausgestaltung der Erfindung ist vorgesehen, dass die Folienbahnen senkrecht zu den Längskanten des Behältnisses mit gegenseitigem Abstand abwechselnd von der einen und der anderen Längskante her streifenförmig miteinander verbunden sind, wobei sich Rippen ausbilden, die von der einen jeweiligen Längskante ausgehend abwechselnd soweit entfernt von der gegenüberliegenden Längskante enden, dass ein Durchlass offen bleibt, der einen mäanderförmigen Strömungsverlauf der abgesaugten Luft erzwingt. Durch das streifenförmige miteinander Verbinden der Folienbahnen entstehen parallele Kammern, durch die die abgesaugte belastete Luft mäanderförmig hindurchströmen muss und die ein flächiges Luftabsaugen ermöglichen.

Das Füllmaterial ist vorzugsweise eine elastische Matte. Das Füllmaterial ist vorzugsweise luftdurchlässig und/oder lässt im Behältnis Gänge frei, die als Luftleitsystem dienen, damit die Luft flächig über den Beutel verteilt abgesaugt werden kann. Auch ist es möglich, dass das Füllmaterial Zonen, die weniger und solche die besser luftdurchlässig sind, aufweist, um so ein Luftleitsystem auszubilden. Ziel des Luftleitsystems ist es, den Luftstrom über die Eintrittsfläche zu verteilen.

Gleichzeitig hält das Füllmaterial die im Wesentlichen luftundurchlässige Oberfläche, z.B. in Form einer oberen und unteren Folienbahn, auf Abstand, vorzugsweise auch dann, wenn das Gewicht des Körpers ganz oder teilweise auf dem Behältnis ruht. Als Füllmaterial sind z.B. Polyesterfasermatten verwendbar, wie sie auch als Grobfiltermatten eingesetzt werden. Die elastischen Matten haben vorzugsweise einen progressiven Tiefenaufbau, d.h. deren Deformationsvermögen ist anfänglich hoch und sinkt mit steigender Kompression. So sind diese anfänglich leicht eindrückbar, lassen sich jedoch nicht über ein bestimmtes Maß unter dem Körpergewicht (z.B. bezogen auf 60 kg) komprimieren, so dass diese auch im komprimierten Zustand luftdurchgängig bleiben. Das Füllmaterial kann nebeneinem Kunststofffaservlies z.B. auch ein geschäumter Kunststoff sein.

Nach einer anderen Ausgestaltung kann das Behältnis der Entlüftungsvorrichtung unter Verwendung von Luftpolsterfolien (auch Noppenfolien oder auf Englisch "bubble wrap" genannt) hergestellt sein. Luftpolsterfolien weisen zumindest eine Deckfolie und einer zweite Lage, in die in regelmäßigen Abständen eine Vielzahl von Luftpolster eingearbeitet ist, auf. Insbesondere wenn die Luftpolsterfolien so zusammengelegt wird, dass die Luftpolster einander zugewandt sind, entsteht ein Innenraum, der auch unter Druckbelastung (wie durch das Körpergewicht eines Liegenden verursacht) Luft leitet. Die Vielzahl der Luftpolster bildet dann das Füllmaterial.

Nach einer weiteren Ausgestaltung hat die Entlüftungsvorrichtung eine langgestreckte Form und ist um den Körper im Bereich der Hüfte im, unter oder auf den Absoprtionsartikel, vorzugsweise unter, legbar, so dass die Zonen der Entlüftungsvorrichtung ohne Durchbrechungen sich im Rückenbereich und die Zonen der Entlüftungsvorrichtung mit Durchbrechungen im Ventralraum auf Höhe des Bauch- oder Unterleibsbereichs, vorzugsweise auf oder unterhalb der Gürtellinie, befinden, wobei der Anschluss im Anfangsbereich und die Zonen der Entlüftungsvorrichtung mit Durchbrechungen im Endbereich der langgestreckten Entlüftungsvorrichtung angeordnet sind. Vorzugsweise hat die gürtelartige Entlüftungsvorrichtung eine Länge von 50%, besonders bevorzugt 65 bis 95% des Körperumfang und eine Breite von 2 bis 12 cm, insbesondere 4 bis 8 cm. Die Entlüftungsvorrichtung dient dann im Rückenbereich als Kühlung. Im Absorptionsartikel kann sich eine Stauwärme entwickeln, insbesondere im Bereich der durch das Körpergewicht druckbelasteten Stellen, die zum Schwitzen und einer zusätzlichen Hautbelastung neigen.

Nach einer weiteren Ausgestaltung der Erfindung ist vorgesehen, dass das Behältnis mit einer luftdurchlässigen Gewebe- oder Vliesschicht umgeben ist, die weiter bevorzugt zusammen mit den Folienbahnen an den Rändern verbunden ist. Die Gewebe- oder Vliesschicht vermeidet den Berührungskontakt der Haut mit der Oberfläche des Behältnisses.

Nach einer weiteren Ausgestaltung der Erfindung ist vorgesehen, dass das Behältnis in seiner Flächenausdehnung an die Gestalt einer Windel oder Binde angepasst ist und in größerer Längenausdehnung um den Körper zumindest teilweise herumlegbar ist. Die Entlüftungsvorrichtung ist insbesondere so gestaltet, dass der Auslass in Form des Anschluss für eine Pumpe nicht so am Behältnis angeordnet ist, dass er unmittelbar auf dem Körper im Auflagebereich des Körpers angeordnet ist, sondern z.B. auf der Bauchseite.

Der Anschluss ist als Sauganschluss für das Absaugen der belasteten Luft aus dem Behältnis und ggf. zusätzlich als Lufteinlass ausgebildet, damit mittels einer Pumpe im Saugbetrieb, die in das Behältnis eingesaugte belastete Luft nach außen abführbar ist und ggf. zeitweilig mittels der Pumpe im Druckbetrieb Luft aus der Umgebung in den Beutel eingeblasen wird und nach außen durch die Durchbrechungen abführbar ist.

Nach einer Ausgestaltung der Erfindung ist vorgesehen, dass der Anschluss der Entlüftungsvorrichtung über eine Schlauchverbindung an eine Pumpe angeschlossen ist. Das Einblasen bzw. Abpumpen von Luft kann in geringen Mengen von beispielsweise 0,1 bis 1 Liter pro Minute, vorzugsweise 0,2 bis 0,5 Liter pro Minute erfolgen.

Dies ist für den Patienten weder mechanisch noch akustisch wahrnehmbar, noch besteht die Gefahr der Unterkühlung. Die Pumpe, in Bezug auf das Arbeitsmedium Luft auch Kompressor oder Verdichter genannt, kann für den Saugbetrieb mit einem Geruchsfilter ausgestattet sein. Als Geruchsfilter eignet sich insbesondere Aktivkohle. Nach einer weiteren Ausgestaltung der Erfindung ist vorgesehen, dass die Saugpumpe in einer schallisolierten, handlichen Box angeordnet ist. Als Pumpe kommt vorteilhafterweise eine Membranpumpe in Betracht. Ein Temperatur- und/oder Feuchtigkeitssensor kann die Pumpe bzw. Pumpenleistung steuern.

Die Entlüftungsvorrichtung kann als separat handhabbare Einheit in den Absorptionsartikel eingesetzt, eingeschoben oder aufgelegt werden. Nach einer weiteren Ausgestaltung der Erfindung ist somit vorgesehen, dass die Entlüftungsvorrichtung im Handel getrennt als Zubehörteil für Einmal- Absorptionsartikel oder in den Absorptionsartikel integriert angeboten wird.

Die Entlüftungsvorrichtung arbeitet in Verbindung mit einem erfindungsgemäßen Verfahren zur Beeinflussung eines feuchtwarmen Klimas im Bereich eines am Körper eines Patienten anliegenden Absorptionsartikels, insbesondere mit Superabsorber, wie beispielsweise einer Windel, und ist gekennzeichnet durch ein Absaugen von feuchtwarmer, belasteter Luft aus dem feuchtwarmen Klimabereich am Patienten mittels einer Entlüftungsvorrichtung, wobei die belastete Luft im feuchtwarmen Klimabereich aufgefangen und aus diesem über die Entlüftungsvorrichtung nach außen abgeführt wird.

In Deutschland gibt es ca. 3 Millionen Menschen die von Dekubitus betroffen sind. Der größte Teil davon ist inkontinent. Die Behandlung oder Vermeidung von Dekubitus erfolgt derzeit mit gepolsterten Matten als Auflagen, oder Wundpflastern und speziellen Verbänden. Bei schwerwiegenden Fällen erfolgt eine Hauttransplantation.

In einer Versuchsreihe wurde die erfindungsgemäße Vorrichtung an einer kleinen Personengruppe unter ärztlicher Aufsicht und Einwilligung der Patienten angewandt.

Die Entlüftungsvorrichtung hatte Schlauchform (ca. 5x85 cm) mit Lufteingang in dem einen Endbereich und Absaugung an dem anderen Ende des Schlauchs und wurde um den Körper herumgelegt in die Windel eingelegt. Luft wurde mit einer Pumpleistung von 0,25 l/min abgesaugt. Der Rückenbereich war frei von Durchbrechungen. Für Neuzugänge mit Inkontinenz aber ohne Dekubitus wurde bei regelmäßiger Anwendung der obigen Vorrichtung bei fast allen Personen die Bildung von Dekubitus verhindert. Dort wo er in seiner schwächsten Form entstand, war eine Behandlung mit Wundpflaster in kurzer Zeit erfolgreich. Bei Patienten mit Dekubitus ergaben sich unerwartet gute Ergebnisse. Im Zusammenwirken mit Wundbehandlungen verkleinerten sich oder schlossen sich Wunden, neue entstanden nicht. Dieses wurde darauf zurückgeführt, dass die feuchte mit Säuren angereicherte Luft im Innenbereich der Windel ständig abgesaugt- und durch frische ersetzt wurde, aber auch darauf, dass die Temperatur im Inneren der Windel und besonders im Rücken durch Kühlung auf ein körpergerechte Temperaturen abgesenkt und das Schwitzen minimiert wurde. Der betreuende Arzt war darüber verblüfft, dass die Vorrichtung in der Lage war vor allem in den Problemzonen wie Kreuzbein und Hüftknochen, Dekubitus zu verhindern und eine Wundheilung auch bei Menschen über 70 Jahren möglich war.

Die Erfindung wird anhand der Zeichnungen näher erläutert. Es zeigen:
Fig. 1 eine Draufsicht auf die beutelartige, flache Entlüftungsvorrichtung, die innerhalb des feucht-warmen Klimas eines Absorptionsartikels, wie einer Windel, angeordnet wird, und mit dessen Hilfe Luft aus dem feucht-warmen Klimabereich abgesaugt und über einen Anschluss nach außen abgeleitet wird,
Fig. 2 eine Ansicht A der Entlüftungsvorrichtung vom Anschluss her gesehen,
Fig. 3 einen vergrößerten Schnitt durch die Entlüftungsvorrichtung längs der Linie III-III nach Fig. 1,
Fig. 4 die Entlüftungsvorrichtung in einer angedeuteten Windelhose mit einem an den Anschluss angeschlossenen Absaugschlauch,
Fig. 5 eine Variante der Entlüftungsvorrichtung in Form einer langgestreckten Beutels, der um den Körper eines Patienten legbar ist und Kammern aufweist, durch die die abgesaugte Luft gezwungen wird, mäanderförmig den Beutel zu durchströmen,
Fig. 6 eine Schnitt-Ansicht B der Entlüftungsvorrichtung nach Fig. 5,
Fig. 7 eine angedeutete Windelhose, in der sich die Entlüftungsvorrichtung nach den Fig. 5 und 6 befindet und die mit der Windelhose um die Hüfte des Patienten gelegt ist, wobei die Hüfte bzw. der Körper des Patienten nicht dargestellt ist, und
Fig. 8 eine durch Spritzguss herstellbare Entlüftungsvorrichtung aus zwei zusammenklappbaren Halbschalen.

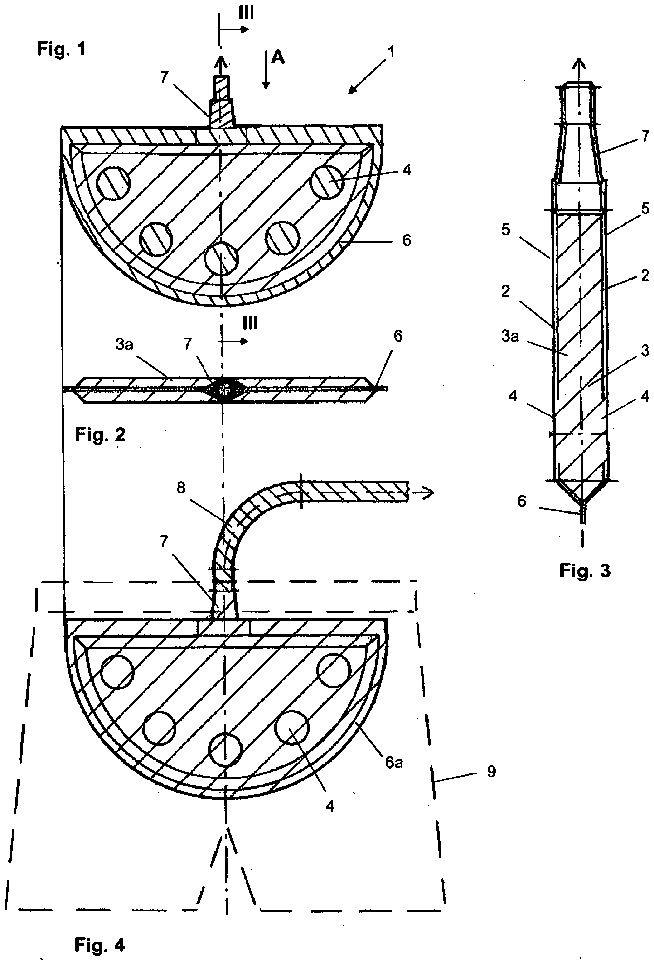

Die Entlüftungsvorrichtung nach den Fig. 1 bis 4 ist ein Behältnis in Form eines Beutels (1) mit einem Auslassstutzen (7) zum Aufsetzen eines flexiblen Schlauchendes. Der Beutel (1) nach Fig. 1 besteht aus zwei Folienbahnen (2). Diese Folienbahnen (2) bilden eine Sperrschicht, die dort, wo sie nicht perforiert ist, den Luftdurchtritt verhindert. Die Folienbahnen (2) werden von einem zwischengefügten Füllstoff (3) auf gegenseitigen Abstand gehalten. Damit ergibt sich im Beutel (1) ein Innenraum (3a). Der Füllstoff (3) ist eine luftdurchlässige, poröse und zusammendrückbare Matte, die aber auch beim Zusammendrücken noch luftdurchlässig bleibt. Die Folienbahnen (2) weisen Durchbrechungen (4) in Form einer Lochperforation auf. Die Gestaltung dieser Lochperforation ist völlig frei. Die Durchbrechungen (4) können auch eckig sein. Der gesamte Beutel (1) ist noch einmal überzogen von einer luftdurchlässigen, hautfreundlichen Vliesschicht (5). An den Rändern (6) sind die Folienbahnen (2) und die Vliesschicht (5) miteinander verschweißt.

An dem Beutel (1) ist ein Absaugstutzen (7) angebracht. An ihn wird ein Absaugschlauch (8) angesetzt, der mit einer nicht dargestellten Absaugpumpe verbunden wird. Diese kann mit Geruchsfilter in einer Box untergebracht sein. Das Absaugvolumen der Luft kann etwa 0,4 Liter pro Minute betragen.

Der Beutel (1) wird entweder als Zubehör angeboten oder er wird gleich bei der Windelherstellung in die Windel eingebaut. Sinn und Zweck des Beutels ist es, aus der Windel belastete Luft abzusaugen aber keine Flüssigkeit. Die belastete Luft wird in den Beutelinnenraum (3a) eingesaugt. Die Pumpe saugt diese, in den Beutelinnenraum (3a) gelangte, belastete Luft über den Schlauchahschluss (7) und den Schlauch (8) nach außen ab. Damit kommt es zu einer Entfeuchtung, vor allem zu einer Abkühlung. So wird einer Entstehung eines Dekubitus entgegengewirkt oder die Behandlung eines bereits bestehenden Dekubitus erleichtert. Der Patient hört die Pumpe und das Strömen der Luft nicht.

In Fig. 4 ist der Beutel (1) in einer solchen Größe dargestellt, dass er in eine Windelhose (9) eingeschoben ist. An den Absaugstutzen (7) ist der Absaugschlauch (8) angeschlossen.

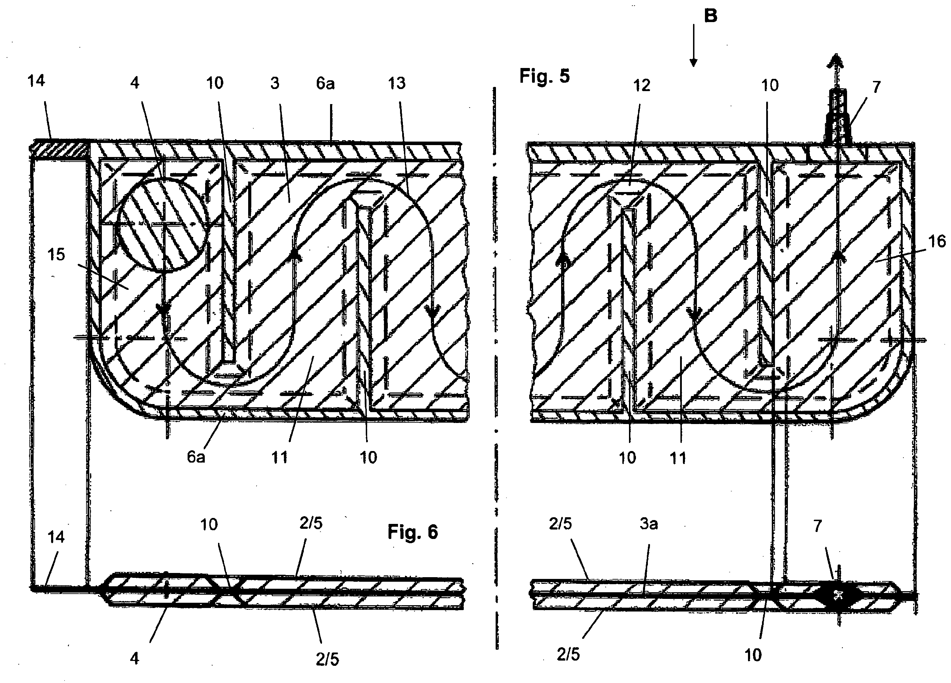

Fig. 5 zeigt eine Variante (1a) des Beutels (1). Dieser Beutel (1a) hat eine solche Länge, dass er um den Körper des Patienten herumgelegt werden kann. Der Beutel (1a) steckt dabei natürlich in der Windel, die eine Windelhose (9) sein kann. Zur besseren Erklärung seines Aufbaues ist der Beutel (1a) abgewickelt dargestellt. Der Beutel (1 a) hat auch in diesem Fall die Gestalt eines flachen Beutels, der allerdings eine Bandform hat. Wie bei den Fig. 1 bis 4 ist ein Füllstoff (3) beidseitig mit Folienbahnen (2) und einem luftdurchlässigen, hautfreundlichen Vlies (5) belegt (Fig. 6). Auf Grund der Bandform ergeben sind parallele Längskanten (6a), die verschweißt oder verklebt sind. Die Folienbahnen (2) wiesen Durchbrechungen (4) auf, die in der ersten vom Auslass am weitesten entfernten Kammer (11) angeordnet sind.

Senkrecht zu den Längskanten (6a) des Beutels (1a) sind die Folienbahnen (2) mit gegenseitigem Abstand abwechselnd von der einen und der anderen Längskante (6a) her streifenförmig miteinander verschweißt oder verklebt. Dabei ergeben sich Rippen (10), die nebeneinanderliegende Kammern (11) bilden. Die von den jeweiligen Längskanten (6a) ausgehenden Rippen (10) sind nicht bis zur jeweiligen Gegenüberkante (6a) durchgezogen. Sie lassen immer einen Durchlass (12) offen. Der über den Absaugstutzen (7) abzusaugenden belasteten Luft wird damit ein mäanderförmiger Strömungsverlauf (13) aufgezwungen. Aus dem ganzen Beutel (1a) wird damit zwangsläufig die eingetretene, belastete Luft abgesaugt.

Mit einem Verschlussband (14) kann der zu einem Ring geformte Beutel (1a) geschlossen werden.

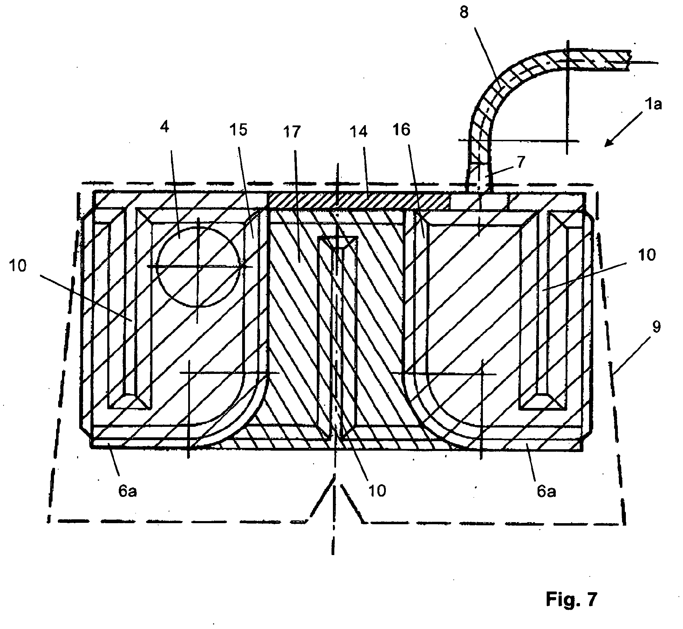

Fig. 7 zeigt den Beutels (1a) in eine Windelhose (9) eingeschoben. Allerdings ist die Windelhose (9) wieder nur in Umrissen und ohne Körper eines Patienten dargestellt. Man sieht vorn liegend das linke und rechte Ende (15, 16) des Beutels (1a). In der Mitte ist der unter dem nicht dargestellten Körper des Patienten befindliche mittlere Teil (17) des Beutels (1a) zu sehen.

Auch der Beutel (1, 1a) wird entweder als Zubehör (1) angeboten oder er wird gleich bei der Windelherstellung in die Windel eingebaut (1a). Sinn und Zweck des Beutels (1a) entspricht dem des Beutels (1) nach den Fig. 1 bis 4. Die Beutel 1 und 1a werden nach dem Gebrauch zusammen mit der Windel entsorgt und können insofern Einmal-Wegwerf-Artikel sein.

Das Arbeitsverfahren zum Abführen der belasteten Luft arbeitet mit den vorstehenden Merkmalen. Der benutzte Beutel (1, 1 a) muss nicht unbedingt in die Absorptionsartikel eingeschoben werden. Er kann auf den Absorptionsartikel aufgelegt oder zwischen Körperhaut und Absorptionsartikel eingelegt sein.

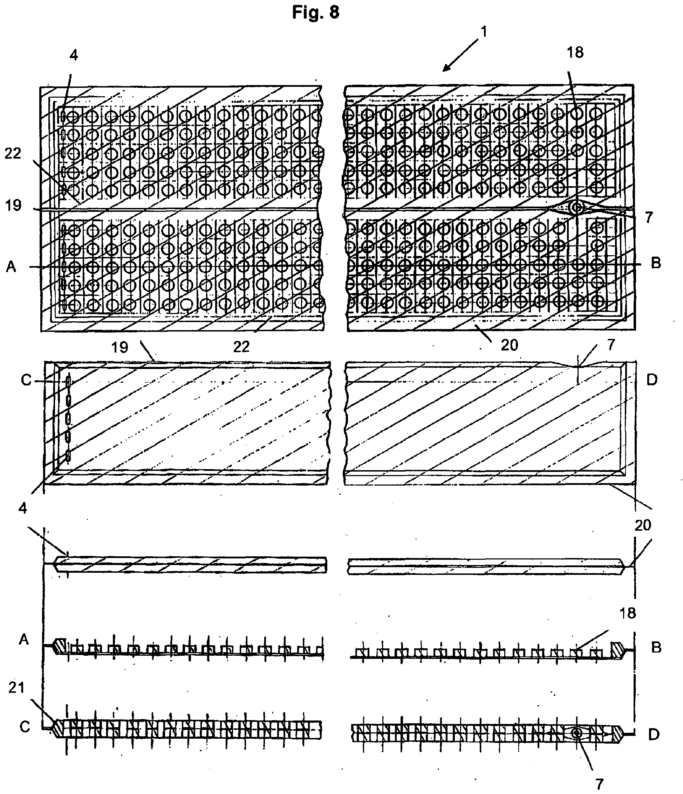

Figur 8 zeigt eine Entlüftungsvorrichtung in mehreren Schnitten, wie Sie durch Spritzguss aus einem flexiblen Kunststoff hergestellt werden kann. Es sind jeweils nur die beiden Endbereiche dargestellt. Oben ist das Behältnis in Form von zwei aufgeklappten Schalen, die über eine Schanierleiste (19) zusammenklappbar sind, dargestellt. Darunter ist die funktionsfähige aus den beiden zusammengeklappten Schalen hergestellte Entlüftungsvorrichtung in Draufsicht und Seitenansicht dargestellt. Eine an die Schanierleiste (19) jeweils anschließende umlaufende Verschlussleiste (20) sichert den Zusammenhalt der beiden Schalen. Im zusammengeklappten Zustand halten gegenüberliegende Distanznoppen (18) die beiden Schalen auf Abstand und geben im Innenraum zwischen den Distanznoppen (18) Luftwege frei. Randleisten unterschiedlicher Höhe (21,22) umschließen den Innenraum dichtend. Der Absaugstutzen (7) ist in dem in Draufsicht rechten Endbereich in die Randleiste 22 eingebracht.

Am anderen Ende finden sich die Durchbrechungen (4) für das Ansaugen der Luft. Dazwischen spannt sich eine nach außen luftundurchlässige Zone auf, die im Rückenbereich auf der Haut anliegen soll. Die flexible Entlüftungsvorrichtung hat eine Länge von 85 cm und ein Breite von 13 cm. Schnitt A-B zeigt einen Schnitt durch eine Schale und Schnitt C-D zeigt einen Schnitt durch beide Schalen im geschlossenen Zustand.

### Bezugszeichenliste:

- (1): Beutel / Behältnis
- (1a): Beutel / Behältnis integriert in die Windel
- (2): Ober- und Unterfolie / Folienbahnen als Wandung des Beutels
- (3): Füllstoff zwischen den Folienbahnen
- (3a): Innenraum von Behältnis oder Beutel
- (4): Durchbrechungen / Perforationslöcher
- (5): luftdurchlässiges Vlies
- (6): verschweißte Ränder
- (6a): verschweißte Längskanten
- (7): Anschluss / Absaugstutzen
- (8): Absaugschlauch
- (9): Absorptionsartikel / Windelhose
- (10): Rippen
- (11): Kammern
- (12): Durchlass
- (13): mäanderförmiger Strömungsverlauf der Luft
- (14): Verschlussband
- (15): Linkes Ende des Beutels (1a)
- (16): Rechtes Ende des Beutels (1 a)
- (17): Rückwärtig gelegenes Mittelteil des Beutels (1a)
- (18): Distanznoppen
- (19): Scharnierleiste
- (20): Verschlusskante
- (21): Randkante
- (22): Halbhohe Randkante

## Patentansprüche

1. Vorrichtung umfassend einen Absorptionsartikel, eine Pumpe und eine Entlüftungsvorrichtung in, unter oder auf dem Absorptionsartikel, wobei
die Entlüftungsvorrichtung ein flexibles Behältnis (1, 1a), einen luftleitenden Innenraum (3a), einen Anschluss (7), und eine Oberfläche (2) mit luftundurchlässigen Zonen und luftdurchlässigen Zonen in Form von Durchbrechungen (4) umfasst, um Luft aus dem Absorptionsartikel durch die Durchbrechungen (4) über den Anschluss in den Innenraum (3a) des Behältnisses (1, 1a) abzusaugen und die Entlüftungsvorrichtung eine separat handhabbare Einheit ist und in den Absorptionsartikel eingeschoben oder ein- bzw. aufgelegt ist,
wobei die Entlüftungsvorrichtung über den Anschluss mit der Pumpe verbunden ist und die Pumpe eine Pumpe im Saugbetrieb ist, damit die belastete Luft aus dem Behältnis über den Anschluss durch Absaugen nach außen abführbar ist.

2. Vorrichtung nach Anspruch 1, wobei die im Wesentlichen luftundurchlässige Oberfläche zumindest eine Ober- und Unterfolie (2) umfasst, die eine Sperrschicht für den Luftdurchgang bilden und zumindest eine der Folien Durchbrechungen (4) für den Luftdurchtritt aufweist.

3. Vorrichtung nach Anspruch 1 oder 2, wobei das Behältnis (1, 1a) mit einer luftdurchlässigen Gewebe- oder Vliesschicht (5) ganz oder teilweise versehen ist, die vorzugsweise die Ober- und Unterfolie (2) überdeckt und weiter bevorzugt zusammen mit der Ober- und Unterfolie (2) an den Rändern (6, 6a) verbunden Ist.

4. Vorrichtung nach einem oder mehreren der vorhergehenden Ansprüche, wobei ein elastischer, luftleitender Füllstoff (3) gegenüberliegende Oberflächen (2) und Insbesondere Ober- und Unterfolie (2) beabstandet.

5. Vorrichtung nach einem oder mehreren der vorhergehenden Ansprüche, wobei die Entlüftungsvorrichtung weiterhin einen Schlauch (8) zur Verbindung von Pumpe und Anschluss (7) aufweist, der mit dem Anschluss (7) am Behältnis (1,1a) koppelbar Ist, wobei unabhängig hiervon die Pumpe vorzugsweise Luft durch einen Geruchsfilter absaugt.

6. Vorrichtung nach einem oder mehreren der vorhergehenden Ansprüche, wobei das Behältnis (1,1a) in seiner Flächenausdehnung an die Gestalt des Absorptionsartikels (9), insbesondere einer Windel, angepasst ist und/oder um den Körper herumlegbar ist.

7. Vorrichtung nach einem oder mehreren der vorhergehenden Ansprüche, wobei das Behältnis (1, 1a) der Entlüftungsvorrichtung benachbarte Längskanten (6a) aufweist, die beabstandet abwechselnd von der einen und der anderen Längskante (6a) her streifenförmig miteinander verbunden sind, wobei sich Rippen (10) ausbilden, die abwechselnd von der einen jeweiligen Längskante (6a) ausgehen und so weit entfernt von der gegenüberliegenden Längskante (6a) enden, dass ein Durchlass (12) offen bleibt, der einen mäanderförmigen Strömungsverlauf (13) der Luft im Innenraum (3b) erzwingt.

8. Vorrichtung nach Anspruch 7, wobei die Randverbindungen (6, 6a) und die Rippen (10), an denen die Folienbahnen (2) - und ggf. auch die darüber befindlichen Gewebe- oder Vliesschichten (5) - miteinander verbunden sind, durch ein Verkleben oder Verschweißen gebildet sind.

9. Vorrichtung nach einem oder mehreren der vorhergehenden Ansprüche, wobei die Entlüftungsvorrichtung in eine Windelhose eingeschoben, zwischen Körperhaut und Absorptionsartikel eingeschoben oder auf den Absorptionsartikel aufgelegt ist.

10. Vorrichtung nach einem oder mehreren der vorhergehenden Ansprüche, wobei der Absorptionsartikel einen Superabsorber umfasst, der polare Flüssigkeiten, wie Wasser oder Urin, aufnimmt.

11. Vorrichtung nach einem oder mehreren der vorhergehenden Ansprüche, wobei die Entlüftungsvorrichtung und der Absorptionsartikel an einen Körper so anlegbar und relativ zueinander angeordnet sind, dass die Durchbrechungen (4) der Entlüftungsvorrichtung sich in Lotrichtung über den Orten wo Stuhlgang und/oder Urin im Absorptionsartikel aufgefangen wird, befinden, um ein Aufsaugen von Flüssigkeit zu verhindern.

12. Vorrichtung nach einem oder mehreren der vorhergehenden Ansprüche, wobei die Entlüftungsvorrichtung keine Flüssigkeiten ansaugt, abgesehen von Kondensat aus der Luft.

13. Vorrichtung nach einem oder mehreren der vorhergehenden Ansprüche, wobei die Entlüftungsvorrichtung
- eine langgestreckte Form hat und um den Körper im Bereich der Hüfte im, unter oder auf den Absorptionsartikel, vorzugsweise unter, legbar ist,
- die Zonen der Entlüftungsvorrichtung ohne Durchbrechungen im Rückenbereich angeordnet sind,
- die Zonen der Entlüftungsvorrichtung mit Durchbrechungen im Ventralraum auf Höhe des Bauch- oder Unterleibsbereichs angeordnet sind, und
- sich der Anschluss (7) im Anfangsbereich und die Zonen der Entlüftungsvorrichtung mit Durchbrechungen im Endbereich der langgestreckten Entlüftungsvorrichtung befinden, wobei
- die Entlüftungsvorrichtung eine Länge von vorzugsweise zumindest 50%, besonders bevorzugt 65 bis 95% des Körperumfangs hat.

14. Vorrichtung nach einem oder mehreren der vorhergehenden Ansprüche, wobei der Absorptionsartikel ein Einmal- Absorptionsartikel oder eine Windel oder eine Binde ist.

15. Arbeitsverfahren zur Beeinflussung des feuchtwarmen Klimas im Bereich eines am Körper eines Patienten anliegenden Absorptionsartikels (9), wie beispielsweise einer Windel, **gekennzeichnet durch** ein Absaugen von feucht-warmer, belasteter Luft aus dem Absorptionsartikel mittels einer als eine separat handhabbare Einheit ausgeführten Entlüftungsvorrichtung im, unter oder auf dem Absorptionsartikel (9) umfassend einen von einem flexiblen Behältnis (1, 1a) gebildeten luftleitenden Innenraum (3a) mit luftundurchlässigen und luftdurchlässigen Zonen und einem Anschluss, wobei belastete Luft aus dem Klimabereich des Absorptionsartikels durch die luftdurchlässigen Zonen in den Innenraum (3a) des Behältnisses (1, 1a) und über den Anschluss mit einer Pumpe im Saugbetrieb abgesaugt wird, damit die belastete Luft aus dem Behältnis über den Anschluss durch Absaugen nach außen abführbar ist.

16. Arbeitsverfahren nach Anspruch 15, wobei die Luft von der Pumpe mit 0,1 bis 1 l/min bei 20°C und 1013 mbar abgesaugt wird.

17. Arbeitsverfahren nach Anspruch 15 oder 16, wobei die Entlüftungsvorrichtung bzw. der Absorptionsartikel welter durch eines oder mehrere der Merkmale der Ansprüche 1 bis 15 gekennzeichnet ist/sind.

## Claims

1. A device comprising an absorption article, a pump, and a venting device in, under or on the absorption article, wherein the venting device comprises a flexible container (1, 1a), an air-conducting inner space (3a), a connection (7), and a surface (2) with air-impermeable zones and air-permeable zones in the form of perforations (4), in order to suction air from the absorption article through the perforations (4) via the connection into the inner space (3a) of the container (1, 1 a), and the venting device is a separately manageable unit and is slid into, or inserted into or placed on the absorption article,
wherein the venting device is connected via the connection to the pump, and the pump is a pump in suction operation, so that the contaminated air can be discharged to the outside from the container via the connection by suctioning.

2. The device according to Claim 1, wherein the substantially air-impermeable surface comprises at least an upper film and lower film (2) which form a barrier layer for the air passage, and at least one of the films has perforations (4) for the air passage.

3. The device according to Claim 1 or 2, wherein the container (1, 1a) is provided entirely or partially with an air-permeable fabric or nonwoven layer (5), which preferably covers the upper film and lower film (2) and which more preferably is connected together with the upper film and lower film (2) at the edges (6, 6a).

4. The device according to one or more of the preceding claims, wherein an elastic, air-conducting filler (3) keeps apart the opposite surfaces (2) and particularly the upper film and lower film (2).

5. The device according to one or more of the preceding claims, wherein, the venting device moreover comprises a tube (8) for the connection of pump and connection (7), tube which can be coupled to the connection (7) on the container (1, 1a), wherein, independently thereof, the pump preferably suctions air through an odour filter.

6. The device according to one or more of the preceding claims, wherein the container (1, 1a), in terms of the surface extent thereof, is adapted to the shape of the absorption article (9), in particular of a diaper, and/or it can be placed around the body.

7. The device according to one or more of the preceding claims, wherein the container (1, 1a) of the venting device comprises adjacent longitudinal edges (6a), which are spaced apart and alternately connected by one and the other longitudinal edge (6a) in the form of strips to one another, wherein ribs (10) are formed, which alternately start from a respective longitudinal edge (6a) and end at a distance from the opposite longitudinal edge (6a) such that a passage (12) remains open which forces a meandering flow pattern (13) of the air in the inner space (3b).

8. The device according to Claim 7, wherein the edge connections (6, 6a) and the ribs (10), on which the film webs (2) - and optionally also the fabric or nonwoven layers (5) located above them - are connected to one another, are formed by gluing or welding.

9. The device according to one or more of the preceding claims, wherein the venting device is slid into diaper pants, between body skin and absorption article, or is placed on the absorption article.

10. The device according to one or more of the preceding claims, wherein the absorption article comprises a superabsorbent which absorbs polar fluids such as water or urine.

11. The device according to one or more of the preceding claims, wherein the venting device and the absorption article can be placed against the body and are arranged relative to one another in such a manner that the perforations (4) of the venting device are located in vertical direction above the sites where stool and/or urine is/are collected in the absorption article, in order to prevent suctioning of fluid.

12. The device according to one or more of the preceding claims, wherein the venting device does not suction fluids, except for condensate from the air.

13. The device according to one or more of the preceding claims, wherein the venting device
- has an elongate shape and can be placed around the body, in the area of the hip, in, under or on the absorption article, preferably under,
- the zones of the venting device without perforations are arranged in the back area,
- the zones of the venting device with perforations are arranged in the ventral space at the level of the belly or abdomen area, and
- the connection (7) is located in the initial area and the zones of the venting device with perforations are located in the end area of the elongate venting device, wherein
- the venting device has a length that is preferably at least 50%, particularly preferably 65 to 95% of the girth of the body.

14. The device according to one or more of the preceding claims, wherein the absorption article is a disposable absorption article or a diaper or a sanitary towel.

15. A method of operation for influencing the humid and warm climate in the area of an absorption article (9) lying on the body of a patient, such as, for example, a diaper, **characterized by** extraction by suction of humid and warm contaminated air from the absorption article by means of a venting device designed as a separately manageable unit in, under or on the absorption article (9) comprising an inner space (3a) designed to conduct air from a flexible container (1, 1a) with air-impermeable and air-permeable zones and a connection, wherein contaminated air is suctioned from the climate area of the absorption article through the air-permeable zones into the inner space (3a) of the container (1, 1a) and via the connection by means of a pump in suction operation, so that the contaminated air can be discharged to the outside from the container via the connection by suctioning.

16. The method of operation according to Claim 15, wherein the air is suctioned by the pump at 0.1 to 1 L/min at 20 °C and at 1013 mbar.

17. The method of operation according to Claim 15 or 16, wherein the venting device and/or the absorption article is/are characterized moreover by one or more of the features of Claims 1 to 15.

## Revendications

1. Dispositif comportant un article absorbant, une pompe et un dispositif d'extraction d'air dans, sous ou sur l'article absorbant, le dispositif d'extraction d'air comprenant un contenant souple (1, 1a), un espace intérieur (3a) conduisant l'air, un raccord (7) et une surface (2) ayant des zones imperméables à l'air et des zones perméables à l'air sous forme d'ouvertures (4) pour aspirer de l'air à partir de l'article absorbant à travers les perforations (4) par le raccord dans l'espace intérieur (3a) du contenant (1, 1a) et le dispositif d'extraction d'air étant une unité manipulable séparément et étant glissé dans ou introduit dans ou placé sur l'article absorbant,
le dispositif d'extraction d'air étant relié à la pompe par le raccord et la pompe étant une pompe dans le mode d'aspiration pour que l'air chargé soit apte à être évacué vers l'extérieur par aspiration à partir du contenant par le raccord.

2. Dispositif selon la revendication 1, dans lequel la surface sensiblement imperméable à l'air comporte au moins un film supérieur et un film inférieur (2) qui forment une couche barrière pour le passage d'air et au moins l'un des films présente des perforations (4) pour le passage d'air.

3. Dispositif selon l'une des revendications 1 ou 2, dans lequel le contenant (1, 1a) comporte totalement ou partiellement une couche de tissu ou de non-tissé (5) perméable à l'air, qui recouvre de préférence le film supérieur et le film inférieur (2) et de manière encore plus préférée est réuni au film supérieur et au film inférieur (2) sur les bords (6, 6a).

4. Dispositif selon l'une ou plusieurs des revendications précédentes, dans lequel une charge (3) conduisant l'air, élastique, espace des surfaces opposées (2) et en particulier le film supérieur et le film inférieur (2).

5. Dispositif selon l'une ou plusieurs des revendications précédentes, dans lequel le dispositif d'extraction d'air présente en outre un tuyau (8) pour relier la pompe et le raccord (7), lequel tuyau est apte à être couplé avec le raccord (7) sur le contenant (1, 1a), où indépendamment la pompe aspire de préférence de l'air à travers un filtre anti-odeurs.

6. Dispositif selon l'une ou plusieurs des revendications précédentes, dans lequel le contenant (1, 1a) est adapté dans sa superficie à la forme de l'article absorbant (9), en particulier d'une couche, et/ou est apte à être enveloppé autour du corps.

7. Dispositif selon l'une ou plusieurs des revendications précédentes, dans lequel le contenant (1, 1a) du dispositif d'extraction d'air présente des bords longitudinaux adjacents (6a), qui sont reliés l'un à l'autre en forme de bande de manière espacée alternativement à partir de l'un et l'autre des bords longitudinaux (6a), des nervures (10) étant formées, lesquelles partent alternativement de l'un des bords longitudinaux (6a) et se terminent de façon suffisamment distante du bord longitudinal opposé (6a) pour qu'un passage (12) demeure ouvert, lequel passage impose un motif d'écoulement sous forme de méandres (13) de l'air dans l'espace intérieur (3b).

8. Dispositif selon la revendication 7, dans lequel les bords réunis (6, 6a) et les nervures (10), auxquels les nappes de film (2) - et le cas échéant aussi les couches de tissu ou de non tissé (5) se trouvant sur celles-ci - sont interconnectés, sont formées par un collage ou soudage.

9. Dispositif selon l'une ou plusieurs des revendications précédentes, dans lequel le dispositif d'extraction d'air est introduit dans une couche-culotte, introduit entre la peau et l'article absorbant ou placé sur l'article absorbant.

10. Dispositif selon l'une ou plusieurs des revendications précédentes, dans lequel l'article absorbant comprend un superabsorbant, qui absorbe des liquides polaires, comme l'eau ou l'urine.

11. Dispositif selon l'une ou plusieurs des revendications précédentes, dans lequel le dispositif d'extraction d'air et l'article absorbant sont aptes à être appliqués sur un corps et disposés l'un par rapport à l'autre de telle sorte que les perforations (4) du dispositif d'extraction d'air se trouvent dans une direction perpendiculaire au-dessus des endroits où des selles et/ou de l'urine sont recueillies dans l'article absorbant, afin d'empêcher une absorption de liquide.

12. Dispositif selon l'une ou plusieurs des revendications précédentes, dans lequel le dispositif d'extraction d'air n'aspire pas de liquides en dehors du condensat provenant de l'air.

13. Dispositif selon l'une ou plusieurs des revendications précédentes, dans lequel le dispositif d'extraction d'air
- a une forme allongée et est apte à être placé autour du corps dans la région de la hanche dans, sous ou sur l'article absorbant, de préférence sous ;
- les zones du dispositif d'extraction d'air sans perforations sont disposées dans le dos ;
- les zones du dispositif d'extraction d'air avec perforations sont disposées dans l'espace ventral au niveau de l'abdomen ou du bas-ventre ; et
- le raccord (7) se trouve dans la région de début et les zones du dispositif d'extraction d'air avec perforations se trouvent dans la région de fin du dispositif d'extraction d'air allongé,
- le dispositif d'extraction d'air ayant une longueur de préférence d'au moins 50 %, de manière particulièrement préférée de 65 à 95 % du tour de taille.

14. Dispositif selon l'une ou plusieurs des revendications précédentes, dans lequel l'article absorbant est un article absorbant jetable ou une couche ou une serviette périodique.

15. Procédé de travail pour influer sur les conditions chaudes et humides dans la zone d'un article absorbant (9) positionné sur le corps d'un patient, comme par exemple une couche, **caractérisé par** une aspiration d'air chargé chaud et humide hors de l'article absorbant au moyen d'un dispositif d'extraction d'air conçu en tant qu'unité manipulable séparément dans, sous ou sur l'article absorbant (9) comportant un espace intérieur (3a) conduisant l'air formé par un contenant souple (1, 1a) avec des zones imperméables à l'air et perméables à l'air et un raccord, l'air chargé étant aspiré de la zone chaude et humide de l'article absorbant à travers les zones perméables à l'air dans l'espace intérieur (3a) du contenant (1, 1a) et par l'intermédiaire du raccord avec une pompe dans le mode d'aspiration, pour que l'air chargé soit apte à être évacué vers l'extérieur par aspiration à partir du contenant par le raccord.

16. Procédé de travail selon la revendication 15, dans lequel l'air est aspiré par la pompe à raison de 0,1 à 1 L/min à 20°C et 1013 mbar.

17. Procédé de travail selon l'une des revendications 15 ou 16, dans lequel le dispositif d'extraction d'air ou l'article absorbant est/sont caractérisé(s) en outre par une ou plusieurs des caractéristiques des revendications 1 à 15.
